# EUROPEAN PATENT SPECIFICATION

(11) **EP 3 684 804 B1**
(45) Date of publication and mention of the grant of the patent: **02.12.2026**
(21) Application number: 18769202.5
(22) Date of filing: 19.09.2018
(51) Int. Cl.: C07K 16/10

(54) **NOROVIRUS ANTIBODIES**
NOROVIRUS NANOANTIKÖRPER
NANOANTICORPS DIRIGÉS CONTRE LE NOROVIRUS

(30) Priority: 20.09.2017 EP 17192166
(43) Date of publication of application: 29.07.2020
(73) Proprietor: Griffith University, Nathan, Queensland 4111 (AU)
(72) Inventor: HANSMAN, Grant, 69117 Heidelberg (DE); KOROMYSLOVA, Anna, 69221 Dossenheim (DE)
(74) Representative: Altmann Stößel Dick Patentanwälte PartG mbB
(86) International application number: PCT/EP2018/075324
(87) International publication number: WO 2019/057755

(56) References cited:
- US-A1- 2017 247 434
- MATHIEU DONDELINGER ET AL: "Understanding the Significance and Implications of Antibody Numbering and Antigen-Binding Surface/Residue Definition", FRONTIERS IN IMMUNOLOGY, vol. 9, 16 October 2018 (2018-10-16), pages 1 - 15, XP055572450, DOI: 10.3389/fimmu.2018.02278
- SALMEN WILHELM ET AL: "A single nanobody neutralizes multiple epochally evolving human noroviruses by modulating capsid plasticity", NATURE COMMUNICATIONS, vol. 14, no. 1, 16 October 2023 (2023-10-16), UK, XP093286197, ISSN: 2041-1723, Retrieved from the Internet <URL:https://www.nature.com/articles/s41467-023-42146-0> DOI: 10.1038/s41467-023-42146-0
- HANSMAN GRANT S. ET AL: "Development of a broad-spectrum therapeutic Fc-nanobody for human noroviruses", JOURNAL OF VIROLOGY, vol. 98, no. 7, 23 July 2024 (2024-07-23), US, XP093286198, ISSN: 0022-538X, DOI: 10.1128/jvi.00707-24
- ANNA D. KOROMYSLOVA ET AL: "ABSTRACT", JOURNAL OF VIROLOGY., vol. 89, no. 5, 17 December 2014 (2014-12-17), US, pages 2718 - 2730, XP055438443, ISSN: 0022-538X, DOI: 10.1128/JVI.03176-14
- CRAWFORD SUE E ET AL: "Mapping Broadly Reactive Norovirus Genogroup I and II Monoclonal Antibodies", CLINICAL AND VACCINE IMMUNO, AMERICAN SOCIETY FOR MICROBIOLOGY, WASHINGTON, DC, US, vol. 22, no. 2, 1 February 2015 (2015-02-01), pages 168 - 177, XP009183338, ISSN: 1556-679X, DOI: 10.1128/CVI.00520-14
- PARKER TRACY DEWESE ET AL: "Identification of genogroup I and genogroup II broadly reactive epitopes on the norovirus capsid", JOURNAL OF VIROLOGY, THE AMERICAN SOCIETY FOR MICROBIOLOGY, US, vol. 79, no. 12, 1 June 2005 (2005-06-01), pages 7402 - 7409, XP002470341, ISSN: 0022-538X, DOI: 10.1128/JVI.79.12.7402-7409.2005
- G. S. HANSMAN ET AL: "Structural Basis for Broad Detection of Genogroup II Noroviruses by a Monoclonal Antibody That Binds to a Site Occluded in the Viral Particle", JOURNAL OF VIROLOGY, vol. 86, no. 7, 1 April 2012 (2012-04-01), pages 3635 - 3646, XP055111167, ISSN: 0022-538X, DOI: 10.1128/JVI.06868-11
- KOROMYSLOVA ANNA D ET AL: "Nanobodies Targeting Norovirus Capsid Reveal Functional Epitopes and Potential Mechanisms of Neutralization", BIOPHYSICAL JOURNAL, vol. 114, no. 3, 2 November 2017 (2017-11-02), XP085340079, ISSN: 0006-3495, DOI: 10.1016/J.BPJ.2017.11.1222

## Description

The present disclosure relates to a binding polypeptide specifically binding to an epitope comprised in an amino acid sequence corresponding to amino acids 250 to 300 of the norovirus genotype II.10 capsid polypeptide, and to a polynucleotide encoding the same. The present disclosure further relates to a composition comprising the aforesaid binding polypeptide and a carrier, and to the binding polypeptide or the composition comprising the same use in diagnosis and/or for use in medicine. Furthermore, the present disclosure relates to kits, devices, vaccines, methods, and uses related to the aforesaid binding polypeptide. The invention is defined by the appended claims. Any reference in the description to methods of treatment or in vivo diagnosis refer to the compounds, pharmaceutical compositions, and medicaments of the present invention for use in methods of treatment of the human or animal body by therapy or for in vivo diagnosis.

Human noroviruses are single-stranded RNA, non-enveloped viruses in the Caliciviridae family and are the most important cause of outbreaks of gastroenteritis. These viruses cannot grow in cell culture, which has hampered vaccine and antiviral development. The genome has three open reading frames (ORF1-3), where ORF1 encodes the non-structural proteins (NS1 to NS7), ORF2 encodes the capsid protein (VP1), and ORF3 encodes a small structural protein. Expression of the capsid protein in insect cells leads to the self-assembly of empty virus-like particles (VLPs) that are morphologically and antigenically similar to native virions >Hansman, G. S., K. Natori, H. Shirato-Horikoshi, S. Ogawa, T. Oka, K. Katayama, T. Tanaka, T. Miyoshi, K. Sakae, S. Kobayashi, M. Shinohara, K. Uchida, N. Sakurai, K. Shinozaki, M. Okada, Y. Seto, K. Kamata, N. Nagata, K. Tanaka, T. Miyamura, and N. Takeda. 2006. Genetic and antigenic diversity among noroviruses. J Gen Virol 87:909-91). Based on the capsid gene sequences, at least five genogroups (GI-GV) have been assigned (Kroneman, A., E. Vega, H. Vennema, J. Vinje, P. A. White, G. Hansman, K. Green, V. Martella, K. Katayama, and M. Koopmans. 2013. Proposal for a unified norovirus nomenclature and genotyping. Arch Virol 158:2059-2068). The genogroups are further subdivided into genotypes and an association between genetic clusters and antigenicity is evident (Hansman et al., loc. cit.).

The X-ray crystal structure of the norovirus GI genotype 1 (GI.1) VLPs showed that VP1 is divided into two domains, shell (S) and protruding (P) (Prasad, B. V., M. E. Hardy, T. Dokland, J. Bella, M. G. Rossmann, and M. K. Estes. 1999. X-ray crystallographic structure of the Norwalk virus capsid. Science 286:287-290). The S domain forms a scaffold surrounding the RNA, whereas the P domain likely contains the determinants for strain diversity. One major structural distinction among these particles is the position of the P domain on the S domain. In the case of GI.1, the P domain is resting on the S domain, whereas in GV.1 and GII.10, the P domains are raised off the S domain by ~15Å. In most noroviruses, the S and P domains are connected by a flexible hinge region, ~10 amino acids long, which allows for the raised P domains, but likely also permits a certain amount of P domain flexibility on the particles (Smith, T. J. 2011. Structural studies on antibody recognition and neutralization of viruses. Curr Opin Virol 1:150-156).

A variety of anti-norovirus antibodies binding to norovirus capsid proteins has been published, e.g. MAb14-1 418, binding to residues 418 to 426 and 526 to 534 (Shiota T et al., J Virol. 2007 Nov;81(22):12298-306); NV23, NS22, and F120, binding to residues 453-472, NS14, binding to residues 473-494, 8C7, binding to residues 451-489 (Crawford SE et al., Clin Vaccine Immunol. 2015 Feb;22(2):168-77; Kou B et al., Clin Vaccine Immunol. 2015 Feb;22(2):160-7), 5B18, binding to residues 433, 496, 533-535 (Hansman GS et al. J Virol. 2012 Apr;86(7):3635-46), NV3901 and NV3912, binding to residues 454-520 (Parker TD et al., J Virol. 2005 Jun;79(12):7402-9), 1B4 and 1F6, binding to residues 45-55 (Yoda T et al., J Clin Microbiol. 2003 Jun;41(6):2367-71), N2C3, binding to residues 55-60 (Li X et al., Virus Res. 2009 Mar;140(1-2):188-93, Li X et al., Virus Res. 2010 Aug;151(2):142-7), an antibody binding residues 52-56 (Gabriel I. Parra et al. PLoS One. 2013; 8(6): e67592), and Nano-85, binding to residues 517-526 (WO 2016/059113).

Nonetheless, little is known about antibody binding epitopes at the structural level. Recently, the first X-ray crystal structure of a norovirus P domain Fab complex was solved (Hansman, G. S., D. W. Taylor, J. S. McLellan, T. J. Smith, I. Georgiev, J. R. Tame, S. Y. Park, M. Yamazaki, F. Gondaira, M. Miki, K. Katayama, K. Murata, and P. D. Kwong. 2012. Structural basis for broad detection of genogroup II noroviruses by a monoclonal antibody that binds to a site occluded in the viral particle. Journal of virology 86:3635-3646; EP2757111A1). Superposition of the P domain Fab complex on the cryo-EM particle indicated the Fab bound to an occluded site on the particle, i.e., hindered by neighboring P domains. However, this broadly reactive monoclonal antibody recognizes a conserved region on the P domain and is used in a commercial diagnostic ELISA detection kit. Moreover, other monoclonal antibodies are thought to bind this occluded region on particles (Parker et al, loc. cit., Prasad et al. loc. cit.; Shiota et al. loc. cit.). This suggested the occluded region was not only immunoreactive, but also immunodominant.

Despite the described progress toward broadly-reactive antibodies, antibodies suitable in preventing norovirus infection and antibodies permitting broad detection of norovirus strains are still needed. There is, thus, a need in the art for improved antibodies solving the problems as described above.

### Problem to be solved

It is therefore an objective of the present invention to provide improved antibodies avoiding the problems as described above.

### Summary of the invention

These problems are solved by the binding polypeptides, polynucleotides, methods, kits, devices, and compositions with the features of the independent claims. Typical embodiments, which might be realized in an isolated fashion or in any arbitrary combination are listed in the dependent claims.

Accordingly, the present invention relates to a binding polypeptide according to claim 1; also disclosed is a polypeptide specifically binding to an epitope comprised in an amino acid sequence corresponding to amino acids 250 to 300 of the norovirus genotype II.10 capsid polypeptide.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. It will be understood that any component defined herein as being included may preferably be explicitly excluded from the claimed invention by way of proviso or negative limitation.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis. Unless otherwise noted, nucleic acid sequences and amino acid sequences are noted according to conventional notation, Thus, nucleic acid sequences noted in the direction 5' to 3', and amino acid sequences in the direction N-terminal to C-terminal.

The term "norovirus" is, in principle, known to the skilled person, and relates to a member of the genetically diverse group of non-enveloped, single-stranded RNA viruses of the Caliciviridae family. Preferably, the norovirus is a genogroup GII or GI norovirus. More preferably, the norovirus is a genogroup GII norovirus. Preferably, the norovirus is a GI.1, GII.2, GII.3, GII.5, GII.6, GII.7, or GII.8. More preferably, the norovirus is norovirus GII.1, GII.4, GII.12, GII.17, or GII.10. In accordance, the term "norovirus genotype II.10" relates to a norovirus of genogroup GII, subgroup 10. Preferably, the norovirus genotype II.10 is the norovirus whose genome sequence was submitted under the designation "Norwalk virus strain Vietnam 026" at Genbank, Acc No. AF504671.2.

The term "polypeptide" is known to the skilled person and relates to a chemical compound comprising, preferably consisting of, amino acids chemically bonded by peptide bonds. Preferably, said amino acids are L-amino acids; more preferably are L-amino acids cotranslationally inserted in a polypeptide. The polypeptide may be synthesized chemically, in vitro, or in a biological expression system, in particular a cell.

The term "norovirus genotype II.10 capsid polypeptide", as used herein, relates to a norovirus capsid polypeptide having the amino acid of Genbank Acc No. AAT12445.1 and SEQ ID NO:1, preferably including sequences essentially identical to the aforesaid sequence. More preferably, the term relates to a norovirus capsid polypeptide having the amino acid of Genbank Acc No. AAT12445.1 and SEQ ID NO:1.

The term "sequence corresponding to amino acids x to y of the norovirus genotype 11.10 capsid polypeptide", preferably, includes sequences from other norovirus strain capsid polypeptides comprising an amino acid sequence corresponding to amino acids x to y of the norovirus genotype 11.10 capsid polypeptide, but differing in primary structure from said polypeptide. Thus, included are, preferably, sequences from muteins having the property of being capsid polypeptides. Preferably, the capsid polypeptide variant comprises a peptide having an amino acid sequence corresponding to an amino acid sequence of 50 to 700, more preferably 50 to 500, consecutive amino acids comprised in a norovirus genotype 11.10 polypeptide as specified above. Moreover, also encompassed are further polypeptide variants of the aforementioned norovirus genotype 11.10 polypeptide. Such polypeptide variants have at least essentially the same biological activity as the specific polypeptides, in particular the activity of assembling into a norovirus capsid under appropriate conditions, in particular in a host cell. Moreover, it is to be understood that a polypeptide variant as referred to in accordance with the present invention shall have an amino acid sequence which differs due to at least one amino acid substitution, deletion and/or addition, wherein the amino acid sequence of the variant is still, preferably, at least 50%, 60%, 70%, 80%, 85%, 90%, 92%, 95%, 97%, 98%, or 99% identical with the amino acid sequence of the specific polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the sequence it is compared to for optimal alignment. The percentage is calculated by determining, preferably over the whole length of the polypeptide, the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Polypeptide variants referred to herein may be species or, preferably, strain or substrain specific homologs, paralogs, or orthologs of the norovirus genotype II.10 polypeptide. Moreover, the polypeptide variants referred to herein include fragments of the specific polypeptides or the aforementioned types of polypeptide variants as long as these fragments and/or variants have the properties as referred to above. Such fragments may be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics. More preferably, the sequence corresponding to amino acids x to y of the norovirus genotype II.10 capsid polypeptide is the sequence of the norovirus genotype II.10 capsid polypeptide, preferably as specified herein below.

The term "epitope" is known to the skilled person as a part of an antigen recognized by at least one component of the immune system. According to the present invention, the antigen is a polypeptide, i.e. a norovirus capsid polypeptide, accordingly, the epitope is a continuous or non-continuous stretch of amino acids specifically bound by the binding polypeptide of the invention. More preferably, the epitope is a continuous stretch of from three to 15, more preferably four to 12, most preferably five to ten amino acids. As will be understood, the binding polypeptide of the present invention may additionally bind to one or more further amino acid(s) of its target; thus, the binding polypeptide may further bind to a second epitope. Preferably, the binding of the binding polypeptide to the first and the second epitope occurs via the same binding site on the binding polypeptide in such case.

Preferably, the epitope is comprised in an amino acid sequence corresponding to amino acids 260 to 280, preferably amino acids 269 to 276, of the norovirus capsid polypeptide. Thus, the epitope is preferably comprised in the amino acid sequence YTNPNESAIVQCQNGRCTLDGELQGTTQLLPTGICAFRGKVTQQVQDEHRG (GII.10 capsid polypeptide fragment 250-300; SEQ ID NO:2). More preferably, the epitope is comprised in the amino acid sequence QCQNGRCTLDGELQGTTQLLP (GII.10 capsid polypeptide fragment 260-280; SEQ ID NO:3). Most preferably, the epitope is comprised in the amino acid sequence DGELQGTT (GII.10 capsid polypeptide fragment 269-276; SEQ ID NO:4). Preferably, the epitope comprises the motif a-x-a-h-x-h-x-o, with "x" being any amino acid; "a" being glutamic acid or aspartic acid; "h" being glycine, alanine, valine, leucine or isoleucine, and "o" being serine or threonine. More preferably, the epitope comprises the motif D-x-E-L-x-G-x-T (SEQ ID NO:6), with "x" being any amino acid. Preferably, the binding peptide further specifically binds to a second epitope comprised in the amino acid sequence corresponding to amino acids 450 to 490, preferably amino acids 470 to 488, of said norovirus genotype II.10 capsid polypeptide. Thus, the second epitope is preferably comprised in the amino acid sequence GGYGNPAIDCLMPQEWVQHLYQESAPSLSDVALVRYVNPET (GII.10 capsid polypeptide fragment 450-490, SEQ ID NO:7), preferably in the amino acid sequence YQESAPSLSDVALVRYVNP (GII.10 capsid polypeptide fragment 470-488, SEQ ID NO:8). Preferably, said second epitope comprises the motif (N or Q)-(D or E), more preferably Q-E. Even more preferably, said second epitope comprises the motif (N or Q)-(D or E)-(x)₁₅-P (SEQ ID NO:9), most preferably Y-Q-E-S-x-P-(x)₁₂-P (SEQ ID NO:10).

The term, "binding polypeptide", as used herein, relates to a polypeptide specifically binding to an epitope comprised in the amino acid sequence corresponding to amino acids 250 to 300 of a norovirus genotype 11.10 capsid polypeptide as specified herein above. Preferably, the binding polypeptide binds to the norovirus polypeptide of the present invention with sufficient affinity to allow detection of a binding polypeptide/norovirus polypeptide complex. Preferably, the dissociation constant (Kd) of the binding polypeptide/norovirus polypeptide complex is at most 10⁻⁷ mol/L, more preferably, at most 10⁻⁸ mol/L, most preferably, at most 10⁻⁹ mol/L. Preferably, the binding polypeptide competes in binding to a capsid polypeptide of a norovirus genogroup II (GII), preferably GII.10, with a VHH comprising the amino acid sequence of SEQ ID NO:11, preferably encoded by SEQ ID NO:12. The binding polypeptide is a single domain antibody that comprises the complementarity determining regions (CDRs) RIIFFMYD (CDR1, SEQ ID NO:13), preferably encoded by SEQ ID NO: 14; QINSDVST (CDR2, SEQ ID NO:15), preferably encoded by SEQ ID NO: 16; and YCNVRRASA (CDR3, SEQ ID NO:17), preferably encoded by SEQ ID NO: 18. As will be understood by the skilled person, the relative positioning of the CDRs with regards to each other and to the structural frame can be determined from SEQ ID NO:11.

The term "specifically binding" is understood by the skilled person. Preferably, specific binding relates to a binding in which the affinity of the binding polypeptide to a norovirus capsid polypeptide, preferably a norovirus polypeptide as specified elsewhere herein, is at least tenfold, preferably at least 100fold, most preferably at least 1000fold higher than for any non-target polypeptide or non-norovirus polypeptide present in a sample. Accordingly, the dissociation constant (Kd) of any binding polypeptide/non-target polypeptide complex is at least 10⁻⁶ mol/L, more preferably, at least 10⁻⁵ mol/l, most preferably, at least 10⁻⁴ mol/L.

Preferably, the binding polypeptide of the present invention is an antibody. The term "antibody" is used herein in the broadest sense and specifically covers monoclonal antibodies, polyclonal antibodies, multispecific antibodies (e.g. bispecific antibodies) formed from at least two intact antibodies, single-domain-antibodies (VHH), also known as nanobodies, and antibody fragments so long as they exhibit the desired binding activity as specified elsewhere herein. Preferably, an antibody is not an antibody naturally comprised in an antiserum, typically not a polyclonal antibody or a polyclonal serum. Preferably, the antibody is a full-length antibody or an antibody fragment. More preferably, the antibody is a monoclonal antibody. Most preferably, the antibody is a VHH (nanobody).

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1, and IgA2. The subunit structures and three-dimensional configurations of different classes of immunoglobulins are well known and described generally in, for example, Abbas et al., Cellular and Mol. Immunology, 4th ed., W.B. Saunders, Co. (2000). An antibody may be part of a larger fusion molecule, formed by covalent or non-covalent association of the antibody with one or more other proteins or peptides.

"Antibody fragments" comprise a portion of an intact antibody, in an embodiment, comprising the antigen-binding region thereof. Examples of antibody fragments include Fab, Fab', F(ab')2, and Fv fragments; diabodies; linear antibodies; single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen. "Fv" is the minimum antibody fragment which contains a complete antigen-binding site. Preferably, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three hypervariable regions (HVRs, also referred to as complementarity determining regions (CDRs)) of each variable domain interact to define an antigen-binding site. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site. The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 0 404 097; WO 1993/01161; Hudson et al., Nat. Med. 9 (2003) 129-134; and Hollinger et al., PNAS USA 90 (1993) 6444-6448. Triabodies and tetrabodies are also described in Hudson et al., Nat. Med. 9 (2003) 129-134.

The term "monoclonal antibody" as used herein refers to an antibody obtained from a population of essentially homogeneous antibodies, i.e., the individual antibodies comprising the population are identical except for possible mutations, e.g., naturally occurring mutations, that may be present in minor amounts. Thus, the modifier "monoclonal" indicates the character of the antibody as not being a mixture of discrete antibodies. Preferably, such a monoclonal antibody includes an antibody comprising a polypeptide sequence that binds a norovirus polypeptide, wherein the target-binding polypeptide sequence was obtained by a process that includes the selection of a single binding polypeptide sequence from a plurality of polypeptide sequences. For example, the selection process can be the selection of a unique clone from a plurality of clones, such as a pool of hybridoma clones, phage clones, or recombinant DNA clones.

The term "single-domain antibody" (VHH) or "nanobody", relates to an antibody fragment comprising one variable antibody domain and is, in principle, known to the skilled person. A review is provided, e.g. in Muyldermanns et al. (2009), Vet Immunol Immunopathol. 128(1-3):178. Preferably, the VHH comprises the CDRs of a heavy-chain antibody, preferably obtained from an alpaca, dromedar, camel, llama, or shark immunized with a target polypeptide. More preferably, the VHH has the binding properties as specified above. Still more preferably, the VHH comprises the complementarity determining regions (CDRs) RIIFFMYD (CDR1, SEQ ID NO:13), preferably encoded by SEQ ID NO: 14; QINSDVST (CDR2, SEQ ID NO:15), preferably encoded by SEQ ID NO: 16; and YCNVRRASA (CDR3, SEQ ID NO:17), preferably encoded by SEQ ID NO: 18. Most preferably, the VHH comprises the amino acid sequence of SEQ ID NO:11, preferably encoded by a polynucleotide comprising the nucleic acid sequence of SEQ ID NO:12.

It is understood by the skilled person that the binding properties of an antibody, in particular of a VHH, usually are conserved if amino acids, in particular those not comprised in a CDR, are exchanged. Accordingly, the term binding polypeptide also relates to polypeptides comprising an amino acid sequence having at least 70 %, more preferably at least 80 %, still more preferably at least 90 %, even more preferably at least 95 %, most preferably at least 99 %, sequence identity to SEQ ID NO:11 and having the property of specifically binding the norovirus polypeptide according to the present invention. More preferably, term binding polypeptide also relates to polypeptides comprising an amino acid sequence having at least 70 % sequence identity to SEQ ID NO:11 and comprising CDR1, CDR2, and/or CDR3 as specified above, preferably located within the sequence at essentially the same position(s) as in SEQ ID NO:11. Such exchanges of amino acids and variants can e.g. be used for providing a humanized binding polypeptide or a binding polypeptide resistant to acid or enzymatic cleavage.

Also, preferably, the binding polypeptide may be, preferably covalently, linked to a chemical molecule. Methods how to covalently link a polypeptide with a chemical molecule are known in the art. Preferred chemical molecules for covalently linking with the binding polypeptide of the present invention are polymers like, e.g. polyethylene glycol, polysaccharides such as starch or one of its derivatives, targeting molecules, and the like.

Moreover, the binding polypeptide may be a fusion polypeptide comprising a further polypeptide portion with a further functionality. E.g. said fusion polypeptide may comprise a linker and/or a tag. As used herein, the term "tag" relates to a detectable chemical or physical moiety covalently connected with the binding polypeptide of the present invention. In an aspect, the tag is an affinity tag or, preferably, a hapten, i.e. a tag having affinity to an affinity compound. In yet a further aspect, the binding of the affinity tag to the affinity compound has a dissociation constant so low that the affinity tag will only dissociate to a negligible extent from the affinity compound. In a yet further aspect, the dissociation constant of the affinity tag:affinity compound pair is less than 10⁻⁵ mol/l (as it is the case with the Strep-Tag:Strep-Tactin binding), less than 10⁻⁶ mol/l (as it is the case in the Strep-TagII:Strep-Tactin binding), less than 10⁻⁷ mol/l (as it is typically the case in antibody:antigen binding), less than 10⁻⁸ mol/l, less than 10⁻¹⁰ mol/l, or less than 10⁻¹² mol/l (as it is the case for the Streptavidin:Biotin binding). E.g., preferably, hapten-conjugated nanobodies may be used for the detection of bound norovirus polypeptide or nanovirus particles, preferably in combination with specific conjugates like peroxidase labeled streptavidin. Methods of determining dissociation constants are well known to the skilled artisan and include, e.g., spectroscopic titration methods, surface plasmon resonance measurements, equilibrium dialysis and the like. Preferably, the affinity tag is a His-tag, Strep-tag, V5-tag, Myc-tag, HA-tag, FLAG-tag, or GST. More preferably, the tag is an enzymatic marker tag, i.e. a tag having an enzymatic activity determinable under appropriate conditions. Suitable enzymatic marker tags are well known in the art and include, e.g. horseradish peroxidase, alkaline phosphatase, luciferase, or beta-galactosidase, and the like. In another aspect, the term tag relates to a fluorescent protein tag. Fluorescent protein tags are well known in the art and include the fluorescent proteins from various organisms, e.g. Aequorea victoria, Verrillofungia concinna, Lobophyllia hemprichii, Goniastrea australensis, Favia favus, and the like, as well as derivatives having a wildtype or a modified excitation- and / or emission-spectrum, like GFP, eGFP, YFP, CFP, or RFP. It is understood by the skilled person that a tag of the present invention is not necessarily restricted to one of the categories as detailed above; e.g. an enzymatic marker tag or a fluorescent protein tag may also be used as an affinity tag, e.g. by using an appropriate antibody recognizing said enzymatic marker tag or fluorescent protein tag. Preferably, the tag is genetically encoded, i.e. the tag is provided as the encoding polynucleotide.

Methods for determining the amount of a fusion polypeptide comprising a tag as described herein are well known in the art and depend on the kind of tag used. In a preferred embodiment, a hapten may be used to either coat a solid phase like magnetic particles or microtiter plates. Preferably, the amount of a tagged cleavage product comprising an enzymatic marker tag is determined by incubating a sample comprising said enzymatic marker tag with an appropriate substrate, the product of which produced by the enzymatic action of said enzymatic marker tag can be determined. Examples of substrate/enzymatic marker pairs include 3,3',5,5'-Tetramethylbenzidine (TMB), 3,3'-Diaminobenzidine (DAB), or 2,2'-Azino-bis(3-ethylbenzothiazoline-6-sulphonic acid) (ABTS) conversion by horseradish peroxidase; 4-Methylumbelliferyl phosphate (4-MUP) or p-Nitrophenyl phosphate conversion by alkaline phosphatase; luciferin conversion by luciferase; or 5-Bromo-4-chloro-indolyl-β-D-galactopyranoside conversion by beta-galactosidase. Also preferably, the amount of a tag comprising a fluorescent protein tag is determined by illuminating said fluorescent protein tag at an appropriate wavelength and determining absorbance or fluorescence of the tag. It is understood by the skilled person that practically any tag can be determined by immunological methods, provided that a specific antibody for said tag is available or can be generated.

Advantageously, it was found in the work underlying the present invention that the binding polypeptide of the present invention has the capacity of broadly recognizing members of the genus norovirus. Accordingly, such an antibody can be used in diagnostic tests screening broadly for norovirus infection. Moreover, said antibody has the potential to be used for inhibiting norovirus infection without the need to know which genotype. Moreover, it was found that the antibody of the present invention can inactivate norovirus particles by binding to the specific site indicated, and thereby prevent further spread of an infection. Advantageously, it is also envisaged to use the binding polypeptide of the present invention for sanitization and disinfection purposes. Also, since nanobodies are easy and cost-effective to produce, this offers the possibility to provide norovirus detection kits at low cost. Moreover, since nanobodies are small and can be applied at high densities onto solid surfaces, the sensitivity of norovirus-assays can be improved. The small size of nanobodies is, preferably, particularly advantageous e.g. in immuno-chromatography, since it allows for applying a high molar concentration of antibody onto a limited space; therefore, preferably, a combination of several nanobodies may be used to increase the range of detectable genotypes while not compromising sensitivity. Furthermore, it was found that the antibody of the present invention synergizes with the antibody of WO 2016/059113 in disassembling norovirus capsids.

The definitions made above apply mutatis mutandis to the following. Additional definitions and explanations made further below also apply for all embodiments described in this specification mutatis mutandis.

The present invention further relates to a polynucleotide according to claim 4; also disclosed is a polynucleotide encoding a binding polypeptide according to the present invention, preferably a polynucleotide which
(i) encodes a binding polypeptide comprising an amino acid sequence of SEQ ID NO:11,
(ii) comprises a nucleotide sequence as shown in SEQ ID NO:12,
(iii) encodes a binding polypeptide comprising an amino acid sequence having at least 70 % sequence identity to SEQ ID NO:11, or/and
(iv) comprises a nucleotide sequence having at least 70 % sequence identity to a nucleotide sequence as shown in SEQ ID NO:12.

The term "polynucleotide", as used in accordance with the present invention, relates to a polynucleotide comprising a nucleic acid sequence which encodes a polypeptide having the biological property of specifically binding to a norovirus polypeptide as specified herein above. Suitable assays for measuring the activities mentioned before are described in the accompanying Examples or in standard literature. A polynucleotide encoding a polypeptide having the aforementioned biological activity has been obtained in accordance with the present invention from an alpaca. The polynucleotide, preferably, comprises the nucleic acid sequence shown in SEQ ID NO:12 encoding the polypeptide having an amino acid sequence as shown in SEQ ID NO:11. It is to be understood that a polypeptide having an amino acid sequence as shown in SEQ ID NO:11 may be also encoded, due to the degenerated genetic code, by other polynucleotides as well. It is also understood that, depending on the specific expression system selected, adaptation of codon usage may improve yields.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences shown in SEQ ID NO:12 by at least one nucleotide substitution, addition and/or deletion whereby the variant nucleic acid sequence shall still encode a polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker from standard text books. A preferred example for stringent hybridization conditions are hybridization conditions in 6 ' sodium chloride/sodium citrate (= SSC) at approximately 45°C, followed by one or more wash steps in 0.2 ' SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 ' SSC (pH 7.2). If organic solvent is present in the abovementioned buffer, for example 50% formamide, the temperature under standard conditions is approximately 42°C. The hybridization conditions for DNA:DNA hybrids are preferably for example 0.1' SSC and 20°C to 45°C, preferably between 30°C and 45°C. The hybridization conditions for DNA:RNA hybrids are preferably, for example, 0.1 ' SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to standard textbooks of molecular biology. Further, variants include polynucleotides comprising nucleic acid sequences which are at least 70%, at least 75%, at least 80%, at least 85%, at least 90%, at least 95%, at least 98% or at least 99% identical to the nucleic acid sequences shown in SEQ ID NO:12. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences shown in SEQ ID NO:11. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981))), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the aforementioned nucleic acid sequences is also encompassed as a polynucleotide of the present invention. The fragment shall encode a polypeptide which still has the activity as specified above. Accordingly, the polypeptide encoded may comprise or consist of the domains of the polypeptide of the present invention conferring the said biological activity. A fragment as meant herein, preferably, comprises at least 60, at least 75, at least 90 or at least 120 consecutive nucleotides of any one of the aforementioned nucleic acid sequences or encodes an amino acid sequence comprising at least 20, at least 25, at least 30, or at least 40 consecutive amino acids of any one of the aforementioned amino acid sequences.

The polynucleotides of the present invention either essentially consist of the aforementioned nucleic acid sequences or comprise the aforementioned nucleic acid sequences. Thus, they may contain further nucleic acid sequences as well. Specifically, the polynucleotides of the present invention may encode fusion proteins wherein one partner of the fusion protein is a polypeptide being encoded by a nucleic acid sequence recited above. Such fusion proteins may comprise as additional part other enzymes of the fatty acid or lipid biosynthesis pathways, polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

The polynucleotide of the present invention shall be provided, preferably, either as an isolated polynucleotide (i.e. isolated from its natural context) or in genetically modified form. The polynucleotide, preferably, is DNA, including cDNA, or RNA. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides.

The present invention also relates to a host cell according to claim 15; also disclosed is a host cell comprising the binding polypeptide according to the present invention or the polynucleotide according to the present invention.

The term "host cell", as used herein, relates to a an eukaryotic or, preferably, prokaryotic cell. Preferably, the cell is a cultured cell of a mammal, more preferably a human cell. More preferably, the cell is a bacterial or fungal cell, still more preferably an Escherichia coli cell.

The present invention further relates to a composition according to claim 5; further disclosed is a composition comprising the binding polypeptide according to any the present invention and a carrier.

The term "composition", as used herein, relates to a mixture of compounds comprising at least the binding polypeptide of the present invention and at least one carrier. The carrier(s) must be acceptable in the sense of being compatible with the other ingredients of the composition and being not deleterious to a potential recipient thereof. The carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The carrier(s) is/are selected so as not to affect the biological activity of the composition. Examples of such diluents are distilled water, physiological saline, Ringer's solutions, dextrose solution, and Hank's solution.

Preferably, the composition is a disinfectant. Thus preferably, the composition is used for inactivating norovirus which may be comprised in a solution or, preferably, on a surface. Preferably, said surface is a body surface, preferably of a subject infected with a norovirus or a subject in contact with such an infected subject. As will be understood, the composition may comprise additional components with disinfectant properties in such case, such as one or more alcohol(s). Preferably, the composition further comprises at least one of a protease and an RNase. Also preferably, the composition further comprises at least one of a detergent and a disinfectant. More preferably, the composition further comprises at least a second binding polypeptide specifically binding to a further epitope comprised in the amino acid sequence of a norovirus capsid polypeptide, more preferably wherein said further epitope is non-identical to the epitope and second epitope as detailed above, and is non-identical to an epitope recognized by the binding polypeptide of the present invention. Even more preferably, the second binding polypeptide specifically binds to the amino acid sequence W-V-N-x-F-Y-x, with x being any amino acid (SEQ ID NO:19). Most preferably, the second binding polypeptide is the binding polypeptide of WO 2016/059113, which is herewith incorporated by reference; in particular is the nanobody nano-85 disclosed therein.

Preferably, the composition is a pharmaceutical composition; thus, preferably, the carrier is a pharmaceutically acceptable carrier. In addition, the pharmaceutical composition or formulation may also include other carriers, adjuvants, or nontoxic, nontherapeutic, nonimmunogenic stabilizers and the like. The binding polypeptide of the present invention can be formulated as pharmaceutically acceptable salts. Acceptable salts comprise acetate, methylester, HCl, sulfate, chloride and the like. The pharmaceutical compositions are, preferably, administered topically or systemically. Suitable routes of administration conventionally used for drug administration are oral, intravenous, or parenteral administration as well as inhalation. Preferably, administration is peroral. However, depending on the nature and mode of action of a compound, the pharmaceutical compositions may be administered by other routes as well. Moreover, the binding polypeptide can be administered in combination with other drugs either in a common pharmaceutical composition or as separated pharmaceutical compositions wherein said separated pharmaceutical compositions may be provided in form of a kit of parts. The binding polypeptide is, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables. Preferably, in case of peroral administration, a preparation comprising an acid-resistant coating is used.

A therapeutically effective dose refers to an amount of the binding polypeptide to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 1 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

The present invention also relates to a binding polypeptide according to the present invention or a composition according to the present invention for use of claim 8 or 9; also disclosed is a binding polypeptide according to the present invention or a composition according to the present invention for use in diagnosis; and/or for use in diagnosing a norovirus-infection in a sample of a subject.

The term "diagnosing", as used herein, refers to assessing the probability according to which a subject is suffering or will suffer from a disease or condition referred to in this specification, in particular norovirus infection. As will be understood by those skilled in the art, such an assessment is usually not intended to be correct for 100% of the subjects to be diagnosed. Preferably, the term requires that a statistically significant portion of subjects can be correctly diagnosed to suffer from the disease or condition. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

The term "subject" as referred to herein encompasses animals which can be infected by a norovirus, preferably mammals, and, more preferably, humans. More preferably, said subject suffers from, is suspected to suffer from, or is at risk to suffer from a norovirus infection. It will be understood that said term, preferably, includes subjects suspected to be asymptomatic norovirus carriers. Subjects which suffer from the said infection can be identified by the accompanying symptoms known for the disease. These symptoms are known in the art and described, e.g., in standard text books of medicine. A subject suspected to suffer from the aforementioned disease may be any apparently healthy subject, e.g., investigated by routine clinical screening, or may be a subject being at risk for developing the aforementioned disease.

The present invention also relates to a binding polypeptide according to the present invention or a composition according to the present invention for use of claim 10 or 11; also disclosed is a binding polypeptide according to the present invention or a composition according to the present invention for use in medicine, and/or for use in treating and/or preventing a norovirus-infection in a subject.

The term "treating" refers to ameliorating the diseases or disorders referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools as described herein above.

The tem "preventing" refers to retaining health with respect to the diseases or disorders referred to herein for a certain period of time in a subject. It will be understood that the said period of time is dependent on the amount of the binding polypeptide which has been administered and individual factors of the subject discussed elsewhere in this specification. It is to be understood that prevention may not be effective in all subjects treated with the compound according to the present invention. However, the tem requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The present disclosure further relates to the use of the binding polypeptide according to the present invention or a composition according to the present invention for diagnosing, treating and/or preventing a norovirus-infection. Moreover, the present disclosure relates to a use of the binding polypeptide according to the present invention for inactivating a norovirus particle.

As used herein, the term "inactivating a norovirus particle" relates to inducing a, preferably irreversible, change in the structure and/or composition of the norovirus particle causing the norovirus particle to become non-infectious and/or replication incompetent. Preferably, inactivating is preventing the norovirus particle from binding to its cognate receptor, preventing the norovirus particle from unpacking its genome, or destroying the structure of the norovirus particle.

The present invention further relates to a method of detecting the presence of a norovirus capsid polypeptide in a sample according to claim 12; further disclosed is a method of detecting the presence of a norovirus capsid polypeptide in a sample, comprising
(a) contacting said sample to the binding polypeptide according to the present invention or a composition according to the present invention,
(b) detecting the amount of binding polypeptide/norovirus capsid polypeptide complexes in said sample, thereby
(c) detecting the presence of norovirus capsid polypeptides in said sample.

The method of detecting the presence of a norovirus capsid polypeptide of the present invention, preferably, is an in vitro method. Moreover, it may comprise steps in addition to those explicitly mentioned above. For example, further steps may relate, e.g., to taking or providing a sample for step a), or contacting the binding polypeptide/norovirus capsid polypeptide complexes from step b) to a detection agent. Moreover, one or more of said steps may be performed by automated equipment. Also, preferably, detecting the presence of a norovirus capsid polypeptide is detecting the presence of norovirus particles. Accordingly, detecting the presence of a norovirus capsid polypeptide in a sample from a subject, preferably, is indicative of a norovirus infection prevalent in said subject. Thus, the method of detecting the presence of a norovirus capsid polypeptide, preferably, is a method of diagnosing a norovirus infection.

The term "sample", as used herein, refers to any sample suspected or known to comprise a norovirus capsid polypeptide. It envisaged according to the present invention that the sample may be, e.g. a food sample, a swab from a surface in a kitchen, or a sample of cell culture supernatant. Preferably, however, the sample is a sample of a body fluid, a sample of separated cells, a sample from a tissue or an organ, or a sample of wash/rinse fluid obtained from an outer or inner body surface of a subject. More preferably, the sample is body fluids like blood, plasma, serum, urine, saliva, or lacrimal fluid. Most preferably, the sample is a stool sample. Samples can be obtained by well known techniques and include, preferably, scrapes, swabs or biopsies. Such samples can be obtained by use of brushes, (cotton) swabs, spatula, rinse/wash fluids, punch biopsy devices, puncture of cavities with needles or surgical instrumentation. Preferably, the sample originates from blood or liver. More preferably, the sample originates from the urogenital tract, the oral cavity, the upper aerodigestive tract and the epidermis. Most preferably, the sample originates from the anal canal. Separated cells and/or cell-free liquids may be obtained from cell culture supernatants, body fluids, or the tissues or organs by separating techniques such as filtration, centrifugation, or cell sorting. Separated cells, preferably, are lysed before being used as samples in the present invention by one of the methods well known to the skilled artisan. It is to be understood that the sample may be further processed in order to carry out the method of the present invention. Preferably, in the method for detecting the presence of a norovirus capsid polypeptide, the sample is a sample suspected to comprise norovirus of at least one of genogroup GI and GII, preferably at least one of GI.1, GI.11, GII.1, GII.2, GII.4 2006, GII.4 2012, GII.10, GII.12, and GII.17.

The term "contacting" as used in the context of the methods of the present invention is understood by the skilled person. Preferably, the term relates to bringing a binding polypeptide of the present invention in physical contact with a sample and thereby, e.g. allowing the sample and the binding polypeptide to interact. Preferably, contacting is performed under conditions allowing stable interaction between a binding polypeptide and a norovirus polypeptide.

The amount of binding polypeptide/norovirus capsid polypeptide can be determined in a sample of a subject by techniques well known in the art, including those described herein above. Depending on the nature of the sample, the amount may be determined by ELISA based techniques or by an immuno-chromatography method. To this end, antibodies or aptamers may be used as detection agents which specifically bind to at least one component of the binding polypeptide/norovirus capsid polypeptide and which, upon binding, can be detected by a detectable label. How such antibodies or aptamers can be generated is known to the skilled person. A detectable label may be covalently or reversibly linked to the antibody or aptamer. A covalently linked label may be a radioactive, fluorophore or chemiluminescent moiety while a reversible label may be a secondary antibody or an aptamer which specifically binds to the detection agent and which upon binding can be used to detect the bound detection agent.

Non-claimed disclosure also relates to a method of treating a norovirus infection in a subject, comprising
(a) contacting said subject to a binding polypeptide according to the present invention or a composition according to the present invention, thereby
(b) treating said norovirus infection in said subject.

The method of treating a norovirus infection , preferably, is an in vivo method. Moreover, it may comprise steps in addition to those explicitly mentioned above. For example, further steps may relate, e.g., to diagnosing a norovirus infection, preferably according to the method of detecting the presence of a norovirus capsid polypeptide of the present invention before step a), or administering a further pharmaceutically active compound to said subject. Moreover, one or more of said steps may be performed by automated equipment.

Contacting a subject with the binding polypeptide, preferably, is administering said binding polypeptide comprised in a composition, preferably a pharmaceutical composition, more preferably as specified herein above.

Further disclosure also relates to a method of preventing a norovirus infection, comprising
(a) contacting an object suspected to comprise a norovirus particle with a binding polypeptide according to the present invention or a composition according to the present invention, thereby
(b) preventing a norovirus infection.

The method of preventing a norovirus infection of the present invention, preferably, is an in vitro method; It may, however, also be performed in vivo, e.g. by prophylactically administering the binding polypeptide of the present invention to a subject. Moreover, it may comprise steps in addition to those explicitly mentioned above. For example, further steps may relate, e.g., to estimating the risk of a subject of becoming infected by a norovirus before step a), or applying further chemical compounds, e.g. disinfectants, to an object. Moreover, one or more of said steps may be performed by automated equipment.

For the method of preventing a norovirus infection by contacting a subject at risk of becoming infected, the definitions provided for the method of treating a norovirus infection in a subject provided herein above apply mutatis mutandis. As will be understood by the skilled person, preventing in such case may also include treating the skin of the hands with the binding polypeptide of the present invention.

For the method of preventing a norovirus infection by contacting object suspected to comprise a norovirus particle, it is envisaged that the binding polypeptide of the present invention is applied to said object in order to inactivate viral particles potentially comprised on its surface or therein. Accordingly, the term "object", as used herein, relates to any physical object suspected to comprise norovirus particles. Preferably, said object is a part of the equipment of a lavatory, e.g. a toilet seat, a toilet bowl, a basin, or a faucet. It is, however, also envisaged that the object is an object in another area at high risk of norovirus spread, e.g. equipment and surfaces in a canteen kitchen.

Further, the present invention relates to a kit according to claim 12; also disclosed is a kit for diagnosing, preventing or/and treating a norovirus infection, comprising the binding polypeptide according to the present invention in a housing.

The term "kit" as used herein refers to a collection of the aforementioned means, e.g., a composition comprising the binding polypeptide of the current invention and / or means for contacting a sample under conditions which allow for forming complexes between said binding polypeptide and a norovirus polypeptide, preferably, provided separately or within a single container. The container, also preferably, comprises instructions for carrying out the method of the present invention. The components of the kit are provided, preferably, in a "ready-to-use" manner, e.g., concentrations are adjusted accordingly, etc. Preferably, the kit further comprises at least a second binding polypeptide specifically binding to a second epitope comprised in the amino acid sequence a norovirus capsid polypeptide, preferably wherein said second epitope is non-identical to the epitope or an epitope recognized by said binding polypeptide. More preferably, the second binding polypeptide specifically binds to the amino acid sequence W-V-N-x-F-Y-x (SEQ ID NO:19) as specified herein above. Also preferably, the kit further comprises at least one of a protease and an RNase, further comprises a pharmaceutically acceptable carrier, and/or further comprises at least one of a detergent and a disinfectant.

Also, the present invention relates to a device according to claim 14; further disclosure relates to a device for diagnosing a norovirus infection, comprising the binding polypeptide according to the present invention and means for determining the amount binding polypeptide/norovirus polypeptide complexes formed in the presence of a sample.

The term "device" as used herein in the context of a device for diagnosing a norovirus infection relates to a system of means comprising at least the means operatively linked to each other as to allow the diagnosis. Preferred means for determining the amount of binding polypeptide/norovirus polypeptide formed in the presence of a sample are well known in the art. How to link the means in an operating manner will depend on the type of means included into the device. Preferably, the means are comprised by a single device in such a case. Said device may accordingly include a detection unit and a computer unit for processing data obtained from the detection unit for determining the amount of binding polypeptide/norovirus polypeptide complex. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician.

The present disclosure further relates to a device for preventing or/and treating a norovirus infection, comprising the binding polypeptide according to the present invention and means for administering the same.

The term "device", as used herein in the context of a device for preventing or/and treating a norovirus infection, relates to a system of means comprising at least the means operatively linked to each other as to allow administration of the compound or of the medicament of the present invention. Preferred means for administering medicaments are well known in the art. How to link the means in an operating manner will depend on the type of means included into the device and on the kind of administration envisaged. Preferably, the means are comprised by a single device in such a case. Said device may accordingly include a delivery unit for the administration of the compound or medicament and a storage unit for storing said compound or medicament until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the compound or medicament of the invention. In another preferred embodiment, the device is an intravenous infusion (IV) equipment comprising the compound or medicament. In another preferred embodiment, the device is an endoscopic device comprising the compound or medicament for flushing a site of norovirus infection. In still another preferred embodiment the device is an inhaler comprising the compound of the present invention, wherein, more preferably, said compound is formulated for administration as an aerosol.

### Figure Legends

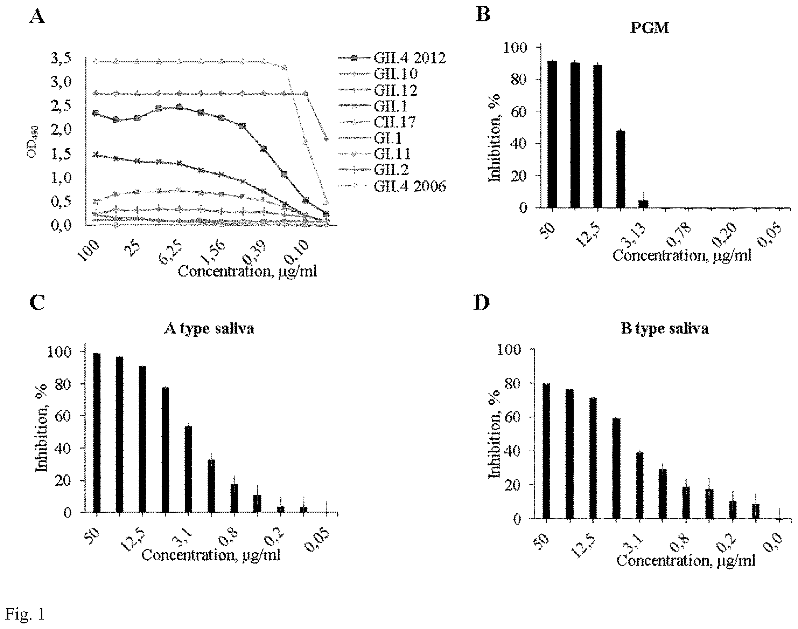

Fig 1. Nano-26 cross-reactivites and inhibition of GII.10 VLP attachment to PGM and saliva. Nanobody cross-reactivities were analyzed using a panel of GII and GI noroviruses in direct ELISA. P domains, 15 µg/ml, (GII.1, GII.2, GII.4, GII.10, GII.12, GII.17) or VLPs, 4 µg/ml, (GI.1 and GI.11) were detected with a panel of serially diluted Nanobody Nano-26. Nano-26 recognized GII.1, GII.2, GII.4, GII.10, GII.12, and GII.17 P domains. All experiments were performed in triplicate (error bars are shown) and the cutoff was set at an OD490 of 0.15 (dashed line). (B) PGM blocking assay was used as a surrogate neutralization assay. GII.10 VLPs were pretreated with serially diluted Nano-26 and added on PGM coated plates. For Nano-26, the IC50 value was 6.6 µg/ml. (C-D) Saliva blocking assay with GII.10 VLPs (2.5 µg/ml) was performed similarly to PGM binding assay with saliva type A (C) or type B (D). Nano-26 inhibited 50% of the binding (IC50) to A type saliva at 2.6 µg/ml, respectively. For B type saliva IC50 values for Nano-26 were 4.3 µg/ml, respectively. Binding was expressed as a percentage of the untreated VLP binding (100%). All experiments were performed in triplicate (error bars are shown).

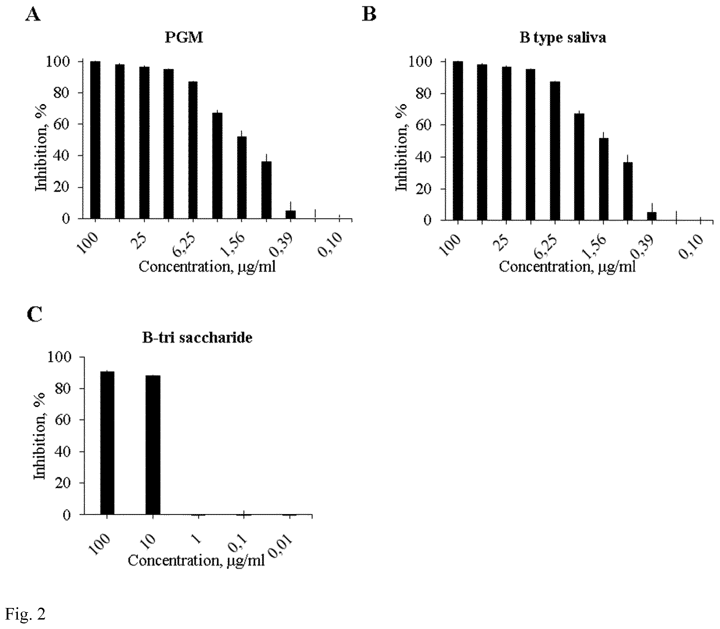

Fig 2. Nano-26 inhibition of GII.4 VLP attachment to PGM, saliva and synthetic HBGAs. (A) Inhibition of GII.4 VLPs (0.5 µg/ml) binding to PGM. Nano-26 blocked GII.4 VLP binding with IC50 of 2.5 µg/ml. (B) Inhibition of GII.4 VLPs (0.5 µg/ml) binding to B type saliva. Nano-26 blocked GII.4 VLP binding with IC50 of 1.2 µg/ml. (C) Inhibition of GII.4 VLPs (0.5 µg/ml) binding to synthetic B-tri saccharide. Both Nano-26 showed a complete inhibition at 10 µg/ml and no inhibition at 1 µg/ml. Binding is expressed as a percentage of the untreated VLP binding (100%). All experiments were performed in triplicate (error bars are shown) and the cutoff was set at an OD490 of 0.15 (dashed line).

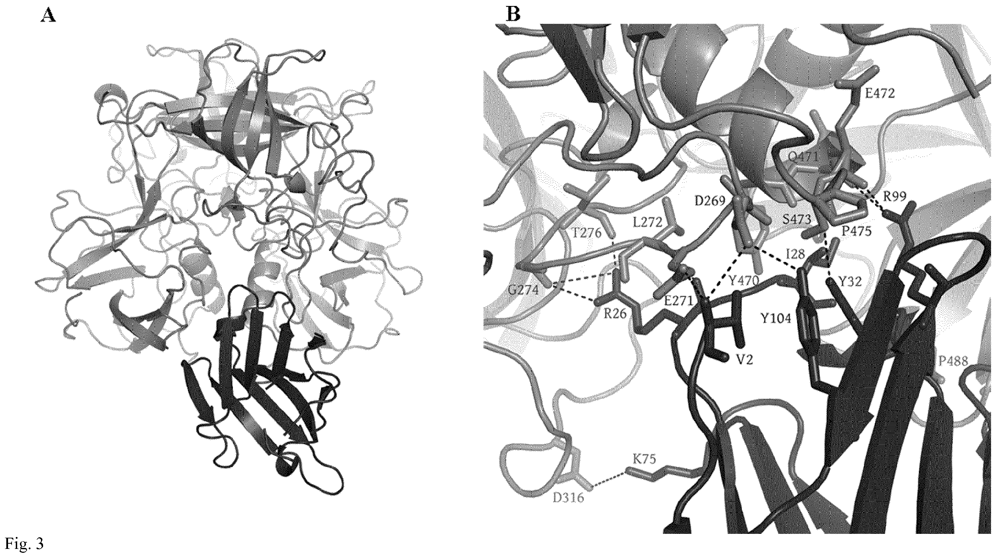

Fig 3. Nanobody treatment of GII.10, GII.4 and GII.17 norovirus VLPs causes capsid deformation. GII.10, GII.4 and GII.17 VLPs were treated with each Nanobody for 1 h at room temperature and applied on EM grids for negative staining. Nano-26 binding caused changes in particle integrity. Negative stain EM images were obtained at 50,000 magnification. The scale bar represents ~50 nm.

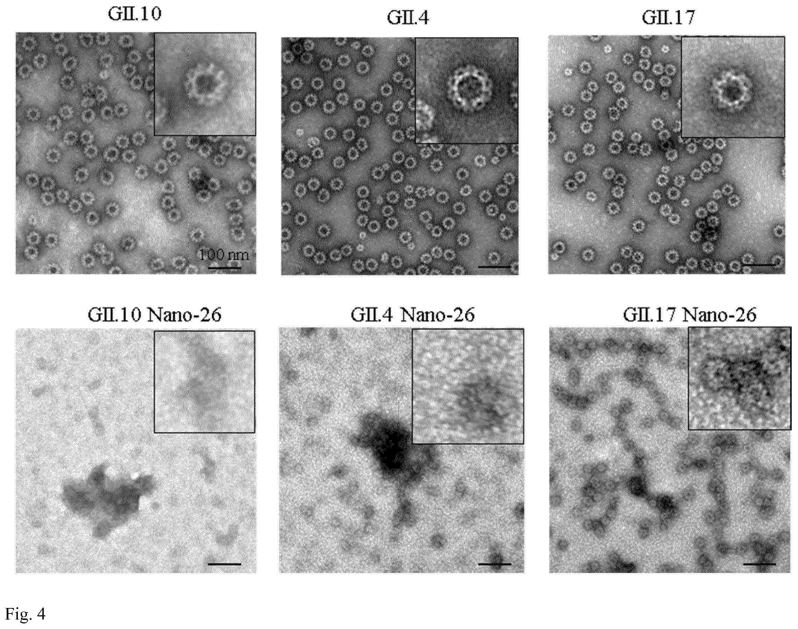

Figure 4. Synergetic effect of Nano-85 and Nano-26. GII.10 VLPs were pre-incubated with Nano-85, Nano-26, or with both Nano-85 and Nano-26 for 30 min at room temperature. After treatment VLPs were subjected to negative staining and examined by EM at 50,000 magnification. VLPs exposed to Nano-85 showed a temperature dependence of morphological changes. At RT small 20-23 nm Nano-85 treated VLPs appeared and prevailed at 37°C. In case of Nano-26 and joint Nano-26 and Nano-85 treatment VLPs were largely degraded at any tested temperature.

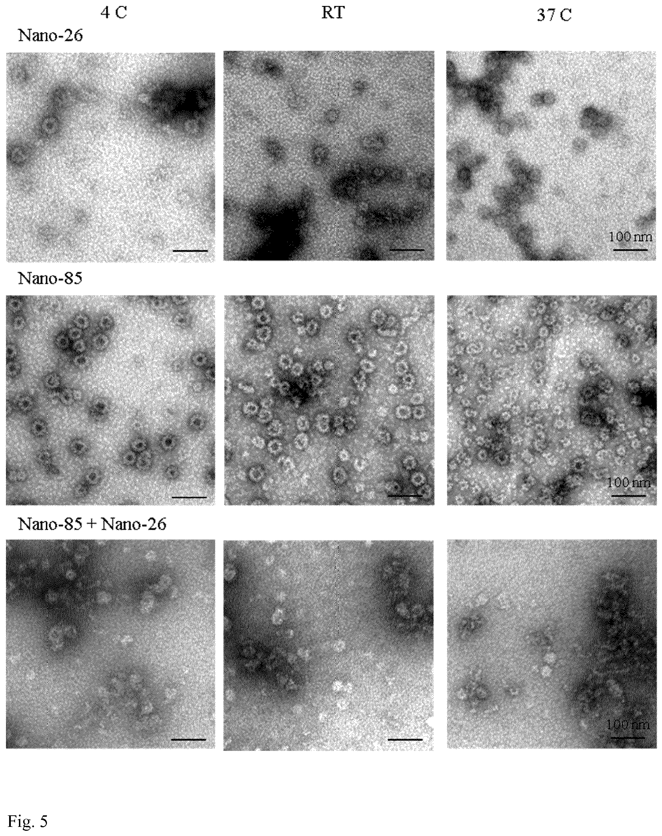

Fig 5. Nano-26 Nano-85 GII.10 P domain double complex structure. GII.10 P domain Nano-26 Nano-85 crystal diffracted to 2.3Å in a C121 space group. Unit cell contained a P domain dimer with two Nano-85 and two Nano-26 molecules. (A) GII.10 P domain is colored as in Fig 3 with Nano-26 (cyan) and Nano-85 (orange). The Nano-85 and Nano-26 binding site in a double complex were identical to binding sites in individual complexes. (B) Nano-26 binds to the cleft between two P domain monomers at the bottom of the P domain dimer. (C) Close up view on the interactions between P domain residues and a Nano-26. Seven direct hydrogen bonds formed between P domain chain B: D269, L272, E274, E471, E472, T276 and Nano-26: V2, R26, R99, and Y104. P domain chain A: 1231, P488 and chain B: E271, D316, Y470, and P475 were involved in hydrophobic interactions and two electrostatic interactions with Nano-26: V2, 128, F30, M31, K75, and A102.

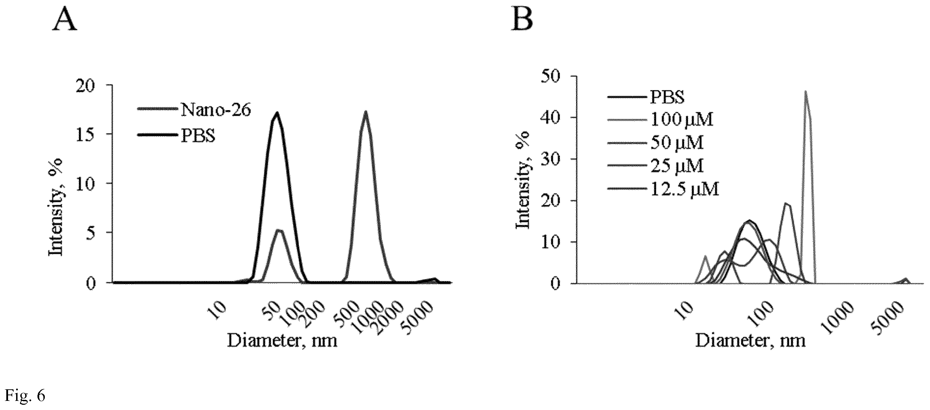

Fig 6. Nanobody treatment leads to changes in norovirus capsid morphology. (A) DLS profiles of Nanobody treated GII.10 VLPs. Nano-26 binding caused the formation of large molecular weight aggregates. All experiments were performed in triplicates. (C) Additionally, GII.4 VLPs were pre-incubated with different concentrations of Nano-26 for one hour at RT. Size distribution was altered at concentrations over 12.5 µM for Nano-26.

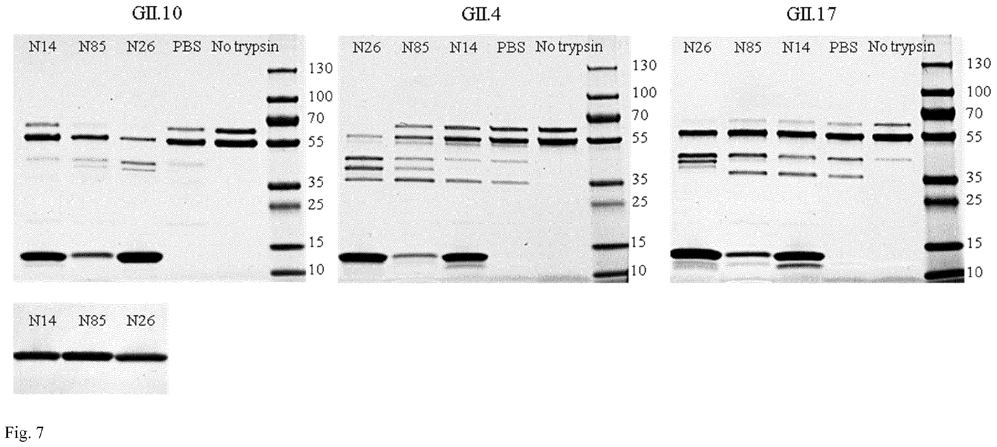

Fig 7. Proteolytic digestion of norovirus VLPs. GII.10, GII.4, and GII.17 VLPs (1 mg/ml) were treated with 1 mg/ml of Nano-26 (lane N26), or PBS for 30 min at 37C. After the treatment samples were exposed to typsin (10 µg/ml final concentration) for additional 30 min at 37C. Samples were then run on the SDS PAGE and stained with Coomassie. VLPs without trypsin cleavage correspond to the lane VLPs. Nano-26 significantly increased the digestion efficiency.

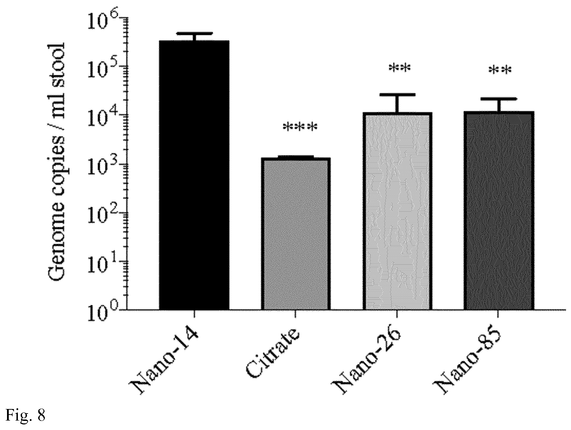

Fig 8. Nano-26 treatment leads to RNA exposure. Concentrated stool suspension was treated with Nano-14, Nano-26, Nano-85, and 250 mM citrate buffer and subsequently with 50 u RNAse One. Genome copies were quantified with RT-qPCR. Nano-26, Nano-85, and citrate caused a significant decrease in genome copy levels compared to Nano-14. Statistical analysis was performed using one-way ANOVA test. Significant differences (P≤0.05) between the treated samples and a negative control (Nano-14 treatment) are marked with stars.

The following Examples shall merely illustrate the invention. They shall not be construed, whatsoever, to limit the scope of the invention.

### Examples

**Example 1: P domain production.** The norovirus P domains, GI.1 (Norwalk virus, Genbank accession number M87661), GI.11 (Akabane, EF547396), GII.1 (Hawaii, U07611), GII.2 (Snow Mountain, AY134748), GII.4 (Sydney-2012, JX459908 and Saga4 2006, AB447457), GII.10 (Vietnam026, AF504671), GII.12 (Hiro, AB044366), and GII.17 (Kawasaki308, LC037415 were expressed in *E.coli,* purified and stored in GFB (25mM Tris-HCl pH7.6, 0.3M NaCl) (Hansman GS, Natori K, Shirato-Horikoshi H, Ogawa S, Oka T, et al. (2006) Genetic and antigenic diversity among noroviruses. J Gen Virol 87: 909-919). The full-length capsid genes, GI.1 (AY502016.1), GI.11, GII.1, GII.2, GII.4, GII.10, GII.12, and GII.17, were expressed in insect cells using the baculovirus expression system and stored in PBS (Hansman GS, Natori K, Oka T, Ogawa S, Tanaka K, et al. (2005) Cross-reactivity among sapovirus recombinant capsid proteins. Arch Virol 150: 21-36; 5. Hansman GS, Saito H, Shibata C, Ishizuka S, Oseto M, et al. (2007) Outbreak of gastroenteritis due to sapovirus. J Clin Microbiol 45: 1347-1349).

**Example 2: Generation of norovirus specific Nanobodies.** Norovirus specific Nanobodies were produced at VIB Nanobody service facility, Belgium as previously described (Koromyslova AD, Hansman GS (2015) Nanobody binding to a conserved epitope promotes norovirus particle disassembly. J Virol 89: 2718-2730). Briefly, a single alpaca was injected with GII.10 VLPs. A VHH library was constructed from isolated peripheral blood lymphocytes and screened for the presence of antigen-specific Nanobodies using phage display. Thirty-five Nanobodies were isolated and allocated to 17 distinct groups based on a sequence alignment. The Nanobody genes were cloned to pHEN6C vector, expressed in WK6 *E.coli* cells, purified and stored in PBS or GFB.

**Example 3: Direct antigen ELISA.** Nanobody titers to norovirus P domains or VLPs were quantified with direct ELISA (17). Briefly, microtiter plates were coated with 7 µg/ml of GII.10 P domains or 2 µg/ml of GII.10 VLPs. For cross-reaction experiments, 15 µg/ml P domain and 4 µg/ml VLPs were coated on ELISA plates. The VLPs or P domain were detected with serially diluted Nanobody and HRP-conjugated mouse α-His-tag monoclonal antibody. Absorbance was measured at 490 nm (OD₄₉₀) and all experiments were performed in triplicate.

**Example 4: Blocking assays.** Pig gastric mucin (PGM) and saliva blocking assays were performed as previously described (Weichert S, Koromyslova A, Singh BK, Hansman S, Jennewein S, et al. (2016) Structural Basis for Norovirus Inhibition by Human Milk Oligosaccharides. J Virol 90: 4843-4848). Briefly, ELISA plates were coated with 10 µg/ml PGM (Sigma, Germany) or with saliva type A or B diluted in PBS 1:2000. Nanobody was two-fold serially diluted in PBS containing 2.5 µg/ml GII.10 VLPs (for PGM assay), 0.5 µg/ml GII.10 VLPs (for saliva assay) or 0.5 µg/ml GII.4 2006 VLPs (both PGM and saliva assay) and incubated for 1 h at RT. The VLPs-Nanobodies mixture was added to the plates and bound VLPs were detected with a α-GII.10 or α-GII.4 VLPs rabbit polyclonal antibody. For synthetic HBGA blocking assay, 10 µg/ml synthetic blood type B trisaccharide amine derivative (Dextra, UK) was coated on Pierce maleic anhydride activated plates (Thermo Fisher Scientific) overnight at 4C. Serially diluted Nanobody was pre-incubated with 5 µg/ml GII.4 VLPs for 1h at RT. Following steps were performed as above. The binding of VLPs-only was set as a reference value corresponding to a 100% binding. The half maximal inhibitory concentrations (IC₅₀) values for Nanobody inhibition were calculated using GraphPad Prism (6.0a).

**Example 5: Isothermal Calorimetry measurements.** Isothermal Calorimetry (ITC) experiments were performed using an ITC-200 (Malvern, UK). Samples were dialyzed into the identical buffer (GFB or PBS) and filtered prior titration experiments. Titrations were performed at 25°C by injecting consecutive (1-2 µl) aliquots of Nano-26 (100-150 µM) into P domain (10-20 µM) with 150 second intervals. The binding data was corrected for the heat of dilution and fit to a one-site binding model to calculate the equilibrium binding constant, K_{A}, and the binding parameters, N and ΔH. Binding sites were assumed to be identical.

**Example 6: P domain and Nanobody complex purification and crystallization.** P domain and Nanobody complexes were purified by size exclusion chromatography (Koromyslova AD, Hansman GS (2015) Nanobody binding to a conserved epitope promotes norovirus particle disassembly. J Virol 89: 2718-2730). The GII.10 P domain Nano-26/Nano-85 complex was crystallized using the following conditions: 0.1 M sodium citrate, 40% (w/v) PEG600. Crystals were grown in a 1:1 mixture of the protein sample and mother liquor at 18°C. Prior to data collection, crystals were transferred to a cryoprotectant containing the mother liquor in 30% ethylene glycol, followed by flash freezing in liquid nitrogen.

**Example 7: Data collection, structure solution, and refinement.** X-ray diffraction data were collected at the European Synchrotron Radiation Facility, France at beamline BM30, ID30A, ID23-1 A and processed with XDS (Kabsch W (2010) XDS. Acta Cryst D66: 125-132). Structures were solved by molecular replacement in PHASER *Phaser-MR* (McCoy AJ G-KR, Adams PD, Winn MD, Storoni LC, Read RJ. (2007) Phaser crystallographic software. Journal of Applied Crystallography 40: 658-674) using GII.10 P domain (PDB ID 3ONU) or GII.17 P domain (5F4M) and a Nano-85 (4X7D) as search models. Structures were refined in multiple rounds of manual model building in COOT (Emsley P LB, Scott WG, Cowtan K. (2010) Features and development of Coot. Acta Crystallographica Section D: Biological Crystallography 66: 486-501) and refined with PHENIX (Adams PD, Afonine PV, Bunkoczi G, Chen VB, Davis IW, et al. (2010) PHENIX: a comprehensive Python-based system for macromolecular structure solution. Acta Crystallographica Section D 66: 213-221). Alternative binding interfaces derived from the crystal packing were analyzed using an online server PDBePISA. The orientation of the Nanobody with the highest interface surface area and contact with CDRs was selected as the biologically relevant interface. Atomic coordinates were deposited to the Protein Data Bank (PDB).

**Example 8: Electron Microscopy and Dynamic Light Scattering.** The norovirus VLP morphology was analyzed using negative stain electron microscopy (EM) as previously described (Koromyslova AD, Hansman GS (2015) Nanobody binding to a conserved epitope promotes norovirus particle disassembly. J Virol 89: 2718-2730). Nanobodies (1 mg/ml) and VLPs (1 mg/ml) were mixed in 1:1 ratio and incubated for 1 h at room temperature. Prior to loading on carbon coated EM grids, all samples were diluted 30 times with distilled water. Grids were washed two times with distilled water and stained with 1% uranyl acetate. The grids were examined on a Zeiss 910 electron microscope (Zeiss, Oberhofen, Germany) at 50,000-fold magnification. VLP diameter was measured with ImageJ software using calibrated pixel/nm scale bar. The hydrodynamic diameters of treated and untreated norovirus VLPs were measured using dynamic light scattering (DLS) on ZetaSizer Nano (Malvern Instruments, UK). Samples were diluted 1:50 with PBS up to a final volume of 1 ml. Three × 12 measurement runs were performed with standard settings (Refractive Index 1.331, viscosity 0.89, temperature 25°C). The average result was created with ZetaSizer software.

**Example 9: Stool treatment and real-time PCR.** In order to determine the effects of the Nanobodies on native virions, we collected GII.4 positive stool samples from two individuals with acute norovirus infection (Doerflinger SY, Weichert S, Koromyslova A, Chan M, Schwerk C, et al. (2017) Human Norovirus Evolution in a Chronically Infected Host. mSphere 2(2):e00352-16). A 10% (w/v) stool suspension was prepared in PBS and clarified by centrifugation at 10,000 × g for 10 min. First stool sample was concentrated by ultracentrifugation at 285000 × g for three hours. Then, 70 µl of the supernatant were treated with 150 µl of each Nanobody (1 mg/ml) for 30 min at room temperature. Samples were digested with 50 units RNAse One (Promega, Germany) for 30 min at 37°C. After treatment total RNA was extracted with QIAamp Viral RNA extraction kit (Qiagen, Hilden, Germany). One step RT-qPCR was performed with previously published GII.4 primers NKP2F (ATG TTY AGR TGG ATG AGA TTC TC), NK2R (TCG ACG CCA TCT TCA TTC AC ) and probe RING2-TP (5'-FAM-TGG GAG GGC GAT CGC AAT CT-TAMRA-3' ) using qScript^{™} XLT One-Step RT-qPCR ToughMix (Quantabio, USA). Viral load was quantified by comparison to a standard curve of GII.4 norovirus RNA transcripts of a known concentration. Average values for three independent experiments are presented. Statistical analysis was performed using one-way ANOVA test. Differences were considered significant when P≤0.05.

**Example 10: Trypsin digestion.** To evaluate the impact of Nanobody binding on capsid susceptibility to proteolytic digestion norovirus VLPs (1 mg/ml) were incubated with Nanobodies (1 mg/ml) in 1:1 ratio for 30 min at 37°C. Then, trypsin-EDTA was added to final concentration of 10 µg/ml for 30 min at 37°C. The concentration of trypsin was chosen to yield only partial cleavage with visible intermediate products. After digestion, samples were loaded on the SDS-12% polyacrylamide gel and stained with coomassie stain.

### Example 11: Binding specificities

The Nanobody binding specificities were initially confirmed with the immunization antigen (i.e., GII.10 VLPs) and the corresponding GII.10 P domain. Nano-26 showed strong binding capacity to both P domain and VLPs (~50 ng/ml). Following these results, the cross-reactivity was analyzed with a panel of VLPs and P domains from various GI (GI.1 and GI.11) and GII (GII.1, GII.2, GII.4 2006 and 2012, GII.10, GII.12, and GII.17) genotypes (Fig 1A). Nano-26 showed broad reactivity, detecting all GII genotypes.

### Example 12: HBGA blocking properties of Nanobodies

In order to determine the HBGA blocking potential of the Nano-26, a surrogate neutralization assays were performed using GII.10 and GII.4 VLPs. Nano-26 inhibited the binding of GII.10 VLPs to PGM in a dose-dependent manner (IC₅₀ = 6.6 µg/ml) (Fig 1B). Similarly, Nano-26 inhibited binding to A-type saliva (IC₅₀ = 3.1 µg/ml) and B-type saliva (IC₅₀ = 4.3 µg/ml) (Fig 1C and 1D). Additionally, Nano-26 blocked GII.4 VLPs from binding to PGM (Fig 2A) (IC₅₀ 2.4 µg/ml) and B-type saliva (IC₅₀ 0.7 µg/ml) (Fig 2B). To demonstrate that Nano-26 specifically inhibit VLP binding to HBGAs present in PGM and saliva, a blocking assay using synthetic HBGAs was performed (Fig 2C). Nano-26 blocked GII.4 VLPs from binding to synthetic B-tri saccharide with IC₅₀ ranging between 1 µg/ml to 10 µg/ml.

### Example 13: Thermodynamic properties

The thermodynamic properties of Nanobody binding to GII.10 P domain were analyzed using ITC. Nano-26 exhibited exothermic binding with nanomolar affinity K_{d} (3.20E-09, M), ΔH (-1.00E+04, cal/mole), ΔS (4.8, cal/mole/deg), ΔG (change in free energy, cal/mol). The binding reaction was driven by a large enthalpy change and was characterized with unfavorable entropy of the binding. This suggested that the net formation of non-covalent bonds between the Nanobody and the P domain was a major contributor to the binding. The stoichiometry indicated the binding of one Nanobody molecule per P domain monomer.

### Example 14: X-ray crystal structures of norovirus P domain Nanobody complex

We solved a double complex structure of GII.10 P domain with Nano-26/Nano-85, which permitted a higher resolution than the GII.10 P domain and Nano-26 complex alone, and explained how two distinct Nanobodies bound simultaneously to one P dimer (Table 1). The overall structure of the P domains in all complex structures was reminiscent of unbound P domain. Nano-26 had typical immunoglobulin fold and interacted with the P domain primarily with CDR loops. The electron density for Nano-26 was well resolved.

**Table 1. Data collection and refinement statistics for P domain Nanobody complex structures.**

| | | | Nano-26 Nano-85 GII.10 P domain |
|---|---|---|---|
| PDB | | | 5O04 |
| Data collection | | | |
| | Space group | | C121 |
| | Cell dimensions | | |
| | | *a, b, c* (Å) | 167.2 91.5 118.1 |
| | | α, β, γ (°) | 90 127.12 90 |
| | Resolution range (Å) | | 47.98-2.30 (2.44-2.30)* |
| | *R*_{merge} | | 7.9 (59.2)* |
| | *I*/*σI* | | 10.7 (2.3)* |
| | Completeness (%) | | 91.7 (90.1)* |
| | Redundancy | | 2.8 (2.7)* |
| | CC1/2 | | 99.6 (76.3) |
| Refinement | | | |
| | Resolution range (Å) | | 47.98-2.30 |
| | No. of reflections | | 58206 |
| | *R*_{work}/*R*_{free} | | 21.2/24.1 |
| | No. of atoms | | 8217 |
| | | Protein | 8064 |
| | | Ligand/ion | 72 |
| | | Water | 81 |
| | Average *B* factors (Å²) | | |
| | | Protein | 52.30 |
| | | Ligand/ion | 56.80 |
| | | Water | 38.20 |
| | RMSD | | |
| | | Bond lengths (Å) | 0.002 |
| | | Bond angles (°) | 0.67 |

| | | | |
|---|---|---|---|
| Each data set was collected from single crystals, respectively. *Values in parentheses are for the highest-resolution shell. | | | |

### Example 15: Structure of GII.10 P domain Nano-26 Nano-85 double complex

Nanobodies were previously shown to aid the crystallization process by increasing protein stability and stabilizing flexible regions (Korotkov KV, Pardon E, Steyaert J, Hol WG (2009) Crystal structure of the N-terminal domain of the secretin GspD from ETEC determined with the assistance of a nanobody. Structure 17: 255-265). We have already utilized Nano-85 to obtain high-resolution complex structures with three different norovirus P domains (Koromyslova AD, Hansman GS (2015) Nanobody binding to a conserved epitope promotes norovirus particle disassembly. J Virol 89: 2718-2730). Herein, we used Nano-85 to improve the resolution of the GII.10 P domain Nano-26 complex structure and describe the synchronized binding of two Nanobodies. The initial structure of GII.10 P domain Nano-26 complex was solved to ~3Å resolution. A single crystal of GII.10 P domain Nano-85/Nano-26 double complex diffracted to 2.3Å in C121 space group. Binding epitopes and interactions of both Nanobodies were identical to those in the individual complexes (ibd.). Nano-26 bound at the bottom of the P domain, perpendicular to Nano-85 binding site (Fig 3).

Nano-26 binding site comprised of residues from both P domain monomers, although the majority of the P domain interactions involved only one chain (chain B). Nano-26 formed seven direct hydrogen bonds with one P domain monomer (chain B: Asp269, Leu272, Gly274, Gln471, Glu472, and Thr276) (Fig 3B). Both P domain monomers were involved in hydrophobic interactions (chain A: Ile231, Pro488; and chain B: Tyr470 and Pro475) with Nano-26. In addition, two electrostatic interactions contributed to the tight binding. Nano-26 binding residues were mainly conserved between GII genotypes, which correlated well with the broad recognition shown with ELISA. Although the binding site was distant from the HBGA binding pocket, Nano-26 had a high inhibition capacity in the blocking assay, which also suggested indirect HBGA interference.

### Example 16: VLP structural integrity upon Nanobody treatment

We previously showed that Nano-85 was able to disassemble norovirus VLPs (Koromyslova AD, Hansman GS (2015) Nanobody binding to a conserved epitope promotes norovirus particle disassembly. J Virol 89: 2718-2730) To explore if these six newly identified Nanobodies had a similar ability, we treated native-size VLPs with Nanobodies and examined the treated-particle morphology using EM. Similarly to Nano-85, Nano-26 treatment partially disassembled and deformed the native-size VLPs (Fig 4). We also performed DLS measurements to quantitatively evaluate GII.10 VLP heterogeneity after Nanobody exposure. Nano-26 treated VLPs mainly formed VP1 protein aggregates, although a small peak corresponding to native-size particles remained. Overall, the DLS analysis corresponded well with the EM results and provided additional evidence that Nanobody treatment altered the capsid structural integrity.

To understand if these effects were relevant for clinically important norovirus strains, GII.4 (Sydney 2012) and GII.17 VLPs were treated with Nano-26 (Fig 4). Nano-26 treatment lead to malformed and aggregated GII.4 VLPs and produced only a few small-size particles. In the case of GII.17 VLPs, caused the formation of small-size VLPs. Overall, these results suggested that effects of the Nanobody treatment might vary among different genotypes. To further evaluate the binding effects of Nano-26 on GII.4 (2012) VLPs, we performed concentration-dependent DLS measurements (S7 and S8 Figs). Nanobody effects were also concentration dependent, with minimum concentrations of 12.5 µM required for Nano-26 (S7C and D Figs). These results suggested that one Nano-26 molecule per VP1 dimer was sufficient to cause morphological changes.

To investigate a temperature dependence of the Nanobody treatment, we mixed GII.10 VLPs with Nano-85 and Nano-26 at 4°C, RT and 37°C for 30 minutes (Fig 5). Nano-85 treated VLPs showed a continuous degradation of native-size particles, producing small and/or partially broken particles as major intermediate forms. Nano-26 was more effective across the temperature range and almost completely altered the VLP integrity. The combination of Nano-85 and Nano-26 appeared to cause a more intense degradation of VLPs.

### Example 17: Nanobody effects on GII.4 virions

In order to examine the Nanobody effects on norovirus virions, we implemented a modified RNA exposure assay and viral loads were quantified using real-time RT-PCR. Concentrated GII.4 positive stool samples were treated with the broadly reactive Nano-26 and Nano-85, while Nano-14 was used as a negative control and 250 mM citric buffer was used as a positive control. Treated samples were then subjected to RNAse One digestion. Nano-26, Nano-85, and citrate treated stool samples showed reduced genome copy numbers compared to the Nano-14 control (approx. 30 times for Nano-26 and Nano-85 and 250 times for citrate) (Fig 10C). These results indicated that the Nano-26 and Nano-85 opened the virions and released the viral RNA, which was degraded by RNAse.

To further investigate if Nanobody treatment could render norovirus VLPs vulnerable to proteolytic cleavage, we subjected GII.10, GII.4, and GII.17 VLPs to a 30-minute trypsin digestion after Nanobody exposure and observed the protein bands using SDS-PAGE (S10 Fig). Nano-14 treated VLPs produced similar bands as the untreated VLPs. Nano-26 and Nano-85 treatment resulted in multiple cleavage products for GII.10 and GII.4 VLPs. In the case of GII.17 VLPs, only Nano-26 treatment showed additional cleavage of the capsid protein. Overall, these results suggested that Nano-85 and Nano-26 caused the particles to become structurally unstable, more vulnerable to proteolytic cleavage, and viral RNA exposure.

Non-standard literature cited in this specification:
Adams PD et al., Acta Crystallographica Section D 2010; 66:213-221
Crawford SE et al., Clin Vaccine Immunol. 2015; 22(2):168-77
Doerflinger SY et al. mSphere 2017; 2(2):e00352-16.
Emsley P et al., Acta Crystallographica Section D: Biological Crystallography 2010: 66:486-501
EP 0 404 097
EP 2 757 111 A1
Gabriel I. Parra et al. PLoS One. 2013; 8(6):e67592
Hansman GS et al. J Virol. 2012; 86(7):3635-46
Hansman, GS et al.. J Gen Virol 2006; 87:909-91
Hansman GS et al. Arch Virol 2005; 150:21-36
Hansman GS et al., J Clin Microbiol 2007; 45:1347-1349.
Hollinger et al., PNAS USA 1993; 90:6444-6448
Hudson et al., Nat. Med. 2003; 9:129-134
Kabsch W, Acta Cryst 2010; D66:125-132
Koromyslova AD & Hansman GS, J Virol 2015; 89:2718-2730.
Korotkov KV et al. Structure 2009; 17:255-265
Kou B et al., Clin Vaccine Immunol. 2015; 22(2):160-7
Kroneman, A et al., Arch Virol 2013; 158:2059-2068
Li X et al., Virus Res. 2009; 140(1-2):188-93
Li X et al., Virus Res. 2010; 151(2):142-7
McCoy AJ et al., Journal of Applied Crystallography 2007; 40: 658-674
Parker TD et al., J Virol. 2005; 79(12):7402-9
Prasad, BV et al., Science 1999, 286:287-290
Shiota T et al., J Virol. 2007; 81(22):12298-306
Smith, TJ. et al, Curr Opin Virol 2011, 1:150-156
Weichert S et al., J Virol 2016; 90: 4843-4848.
WO 1993/01161
WO 2016/059113
Yoda T et al., J Clin Microbiol. 2003;41(6):2367-71.

## Claims

1. A binding polypeptide that specifically binds to an epitope comprised in an amino acid sequence corresponding to amino acids 250 to 300 of the norovirus genotype 11.10 capsid polypeptide,
wherein said binding polypeptide is a single domain antibody that comprises the complementarity determining regions (CDRs) of CDR1: SEQ ID NO:13, CDR2: SEQ ID NO:15, and CDR3: SEQ ID NO:17.

2. The binding polypeptide of claim 1, wherein said binding polypeptide comprises an amino acid sequence having at least 70 % sequence identity to SEQ ID NO:11.

3. The binding polypeptide of claim 1 or claim 2, wherein said binding polypeptide comprises an amino acid sequence having the amino acid sequence of SEQ ID NO:11.

4. A polynucleotide encoding the binding polypeptide according to any one of claims 1 to 3.

5. A composition, preferably a pharmaceutical composition, comprising the binding polypeptide according to any one of claims 1 to 3 and a carrier.

6. The composition of claim 5, wherein said composition further comprises at least a second binding polypeptide specifically binding to a second epitope comprised in the amino acid sequence of a norovirus capsid polypeptide.

7. The composition of claim 6, wherein said second binding polypeptide specifically binds to the amino acid sequence W-V-N-x-F-Y-x, with "x" being any amino acid.

8. A binding polypeptide according to any one of claims 1 to 3 or a composition according to any one of claims 5 to 7 for use in in vivo diagnosis.

9. Use of a binding polypeptide according to any one of claims 1 to 3 or a composition according to any one of claims 5 to 7 for in vitro diagnosing a norovirus-infection in a sample of a subject.

10. A binding polypeptide according to any one of claims 1 to 3 or a composition according to any one of claims 5 to 7 for use in medicine.

11. A binding polypeptide according to any one of claims 1 to 3 or a composition according to any one of claims 5 to 7 for use in treating and/or preventing a norovirus-infection in a subject.

12. A method of detecting the presence of a norovirus capsid polypeptide in a sample, comprising
(a) contacting said sample to the binding polypeptide according to any one of claims 1 to 3 or a composition according to any one of claims 5 to 7,
(b) detecting the amount of binding polypeptide/norovirus capsid polypeptide complexes in said sample, thereby
(c) detecting the presence of norovirus capsid polypeptides in said sample.

13. A kit suitable for diagnosing, preventing or/and treating a norovirus infection, comprising the binding polypeptide of any one of claims 1 to 3 in a housing.

14. A device suitable for diagnosing a norovirus infection, comprising the binding polypeptide of any one of claims 1 to 3 and means for determining the amount of binding polypeptide/norovirus capsid polypeptide complexes formed in the presence of a sample.

15. A host cell comprising the binding polypeptide according to any one of claims 1 to 3 or the polynucleotide according to claim 4.

## Patentansprüche

1. Bindungspolypeptid, das spezifisch an ein Epitop bindet, das in einer Aminosäuresequenz enthalten ist, die den Aminosäuren 250 bis 300 des Norovirus-Genotyp-II. 10-Kapsidpolypeptids entspricht,
wobei das Bindungspolypeptid ein Einzeldomänenantikörper ist, der die komplementaritätsbestimmenden Regionen (CDRs) von CDR1: SEQ ID NO: 13, CDR2: SEQ ID NO: 15, und CDR3: SEQ ID NO: 17 umfasst.

2. Bindungspolypeptid nach Anspruch 1, wobei das Bindungspolypeptid eine Aminosäuresequenz mit mindestens 70 % Sequenzidentität zu SEQ ID NO: 11 umfasst.

3. Bindungspolypeptid nach Anspruch 1 oder Anspruch 2, wobei das Bindungspolypeptid eine Aminosäuresequenz mit der Aminosäuresequenz von SEQ ID NO: 11 umfasst.

4. Polynukleotid, das für das Bindungspolypeptid nach einem der Ansprüche 1 bis 3 codiert.

5. Zusammensetzung, vorzugsweise pharmazeutische Zusammensetzung, umfassend das Bindungspolypeptid nach einem der Ansprüche 1 bis 3 und einen Träger.

6. Zusammensetzung nach Anspruch 5, wobei die Zusammensetzung ferner mindestens ein zweites Bindungspolypeptid umfasst, das spezifisch an ein zweites Epitop bindet, das in der Aminosäuresequenz eines Norovirus-Kapsidpolypeptids enthalten ist.

7. Zusammensetzung nach Anspruch 6, wobei das zweite Bindungspolypeptid spezifisch an die Aminosäuresequenz W-V-N-x-F-Y-x bindet, wobei "x" eine beliebige Aminosäure ist.

8. Bindungspolypeptid nach einem der Ansprüche 1 bis 3 oder Zusammensetzung nach einem der Ansprüche 5 bis 7 zur Verwendung in der In-vivo-Diagnose.

9. Verwendung eines Bindungspolypeptids nach einem der Ansprüche 1 bis 3 oder einer Zusammensetzung nach einem der Ansprüche 5 bis 7 zur In-vitro-Diagnose einer Norovirusinfektion in einer Probe eines Individuums.

10. Bindungspolypeptid nach einem der Ansprüche 1 bis 3 oder Zusammensetzung nach einem der Ansprüche 5 bis 7 zur Verwendung in der Medizin.

11. Bindungspolypeptid nach einem der Ansprüche 1 bis 3 oder Zusammensetzung nach einem der Ansprüche 5 bis 7 zur Verwendung bei der Behandlung und/oder Prävention einer Norovirusinfektion bei einem Individuum.

12. Verfahren zum Nachweisen des Vorhandenseins eines Norovirus-Kapsidpolypeptids in einer Probe, umfassend
(a) Inkontaktbringen der Probe mit dem Bindungspolypeptid nach einem der Ansprüche 1 bis 3 oder einer Zusammensetzung nach einem der Ansprüche 5 bis 7,
(b) Nachweisen der Menge an Bindungspolypeptid/Norovirus-Kapsidpolypeptid-Komplexen in der Probe und dadurch
(c) Nachweisen des Vorhandenseins von Norovirus-Kapsidpolypeptiden in der Probe.

13. Kit, das zur Diagnose, Prävention oder/und Behandlung einer Norovirusinfektion geeignet ist, umfassend das Bindungspolypeptid nach einem der Ansprüche 1 bis 3 in einem Gehäuse.

14. Vorrichtung, die zum Diagnostizieren einer Norovirusinfektion geeignet ist, umfassend das Bindungspolypeptid nach einem der Ansprüche 1 bis 3 und Mittel zum Bestimmen der Menge an Bindungspolypeptid/Norovirus-Kapsidpolypeptid-Komplexen, die in Gegenwart einer Probe gebildet werden.

15. Wirtszelle, umfassend das Bindungspolypeptid nach einem der Ansprüche 1 bis 3 oder das Polynukleotid nach Anspruch 4.

## Revendications

1. Polypeptide de liaison qui se lie spécifiquement à un épitope compris dans une séquence d'acides aminés correspondant aux acides aminés 250 à 300 du polypeptide de capside de norovirus du génotype II.10,
ledit polypeptide de liaison étant un anticorps à un domaine unique qui comprend les régions de détermination complémentaires (CDR) de CDR1 : SEQ ID NO : 13, CDR2 : SEQ ID NO : 15, et CDR3 : SEQ ID NO : 17.

2. Polypeptide de liaison selon la revendication 1, ledit polypeptide de liaison comprenant une séquence d'acides aminés ayant au moins 70 % d'identité de séquence avec la SEQ ID NO : 11.

3. Polypeptide de liaison selon la revendication 1 ou la revendication 2, ledit polypeptide de liaison comprenant une séquence d'acides aminés ayant la séquence d'acides aminés de SEQ ID NO : 11.

4. Polynucléotide codant pour le polypeptide de liaison selon l'une quelconque des revendications 1 à 3.

5. Composition, de préférence composition pharmaceutique, comprenant le polypeptide de liaison selon l'une quelconque des revendications 1 à 3 et un véhicule.

6. Composition selon la revendication 5, ladite composition comprenant en outre au moins un deuxième polypeptide de liaison se liant spécifiquement à un deuxième épitope compris dans la séquence d'acides aminés d'un polypeptide de capside de norovirus.

7. Composition selon la revendication 6, dans laquelle le deuxième polypeptide de liaison se lie spécifiquement à la séquence d'acides aminés W-V-N-x-F-Y-x, « x » étant un acide aminé quelconque.

8. Polypeptide de liaison selon l'une quelconque des revendications 1 à 3 ou composition selon l'une quelconque des revendications 5 à 7 pour l'utilisation dans le diagnostic in vivo.

9. Utilisation d'un polypeptide de liaison selon l'une quelconque des revendications 1 à 3 ou composition selon l'une quelconque des revendications 5 à 7 pour le diagnostic in vitro d'une infection par un norovirus dans un échantillon d'un sujet.

10. Polypeptide de liaison selon l'une quelconque des revendications 1 à 3 ou composition selon l'une quelconque des revendications 5 à 7 pour l'utilisation en médecine.

11. Polypeptide de liaison selon l'une quelconque des revendications 1 à 3 ou composition selon l'une quelconque des revendications 5 à 7 pour l'utilisation dans le traitement et/ou la prévention d'une infection par un norovirus chez un sujet.

12. Méthode de détection de la présence d'un polypeptide de capside de norovirus dans un échantillon, comprenant
(a) la mise en contact dudit échantillon avec le polypeptide de liaison selon l'une quelconque des revendications 1 à 3 ou une composition selon l'une quelconque des revendications 5 à 7,
(b) la détection de la quantité de complexes de polypeptide de liaison/polypeptide de capside de norovirus dans ledit échantillon, ce qui permet
(c) la détection de la présence de polypeptides de capside de norovirus dans ledit échantillon.

13. Kit approprié pour le diagnostic, la prévention et/ou le traitement d'une infection par un norovirus, comprenant le polypeptide de liaison selon l'une quelconque des revendications 1 à 3 dans un boîtier.

14. Dispositif approprié pour le diagnostic d'une infection par un norovirus, comprenant le polypeptide de liaison selon l'une quelconque des revendications 1 à 3 et des moyens pour déterminer la quantité de complexes de polypeptide de liaison/polypeptide de capside de norovirus formés en présence d'un échantillon.

15. Cellule hôte comprenant le polypeptide de liaison selon l'une quelconque des revendications 1 à 3 ou le polynucléotide selon la revendication 4.
